# FASCICULE DE BREVET EUROPEEN

(11) **EP 1 890 614 B1**
(45) Date de publication et mention de la délivrance du brevet: **21.09.2011**
(21) Numéro de dépôt: 05742131.5
(22) Date de dépôt: 01.06.2005
(51) Int. Cl.: A61B 17/16

(54) **INSTRUMENT PORTE-OUTIL**
WERKZEUGHALTERINSTRUMENT
TOOL-HOLDER INSTRUMENT

(43) Date de publication de la demande: 27.02.2008
(73) Titulaire: Inomed Technology SA Courtelary, 2608 Courtelary (CH)
(72) Inventeur: BRUNNER, Raphaël, CH-2502 Biel (CH)
(74) Mandataire: P&TS Patents & Technology Surveys SA
(86) Numéro de dépôt international: PCT/CH2005/000309
(87) Numéro de publication internationale: WO 2006/128310

(56) Documents cités:
- WO-A-99/21686
- US-A- 4 055 185
- US-A1- 2005 159 751

## Description

La présent invention concerne un instrument porte-outil en forme de poignée longitudinale, notamment pour applications chirurgicales, avec un arbre d'entraînement pour un outil, cet arbre pouvant être glissé le long d'un axe longitudinal dans l'instrument à partir de l'extrémité arrière de l'instrument pour être connecte à la partie porte-outil d'un outil rotatif logée dans une tète porte-outil montée à l'extrémité avant de l'instrument.

Il existe de nombreuses constructions d'instruments porte-outil, comme par exemple des perceurs, notamment utilisés dans la chirurgie orthopédique. Certains instruments présentent des têtes par lesquelles les outils (perçage, fraisage, taraudage, alésage) sont orientes sous un angle défini par rapport à l'axe longitudinal de l'instrument.

WO99/21686 décrit un dispositif de support de mandrin qui dispositif est conçu pour être utilisé surtout par des dentistes. Trois éléments cylindriques sont disposés bout à bout de façon à ce que, grâce à l'inclinaison des faces desdits éléments, on peut régler toutes les positions angulaires de 0° à 90°. Deux arbres, un arbre moteur et une transmission flexible, sont nécessaires parce que le deuxième arbre, étant flexibles, peut mieux s'adapter aux mouvements relatives des trois éléments. Lesdits arbres ne sont pas glissés le longe de l'axe longitudinale du dispositif et ne peuvent pas être démontés de façon a ce que un nettoyage efficace puisse être fait.

Un but de la présente invention est de prévoir un instrument porte-outil qui soit utilisable sous des rayons d'action de travail variés dans une multitude de techniques, notamment techniques opératoires de type orthopédiques.

Pour atteindre ce but et résoudre les problèmes relatifs à un instrument porte-outil du type défini ci-dessus, l'instrument présente selon la présente invention les caractéristiques selon la partie caractéristique de la revendication 1.

Des formes d'exécution particulières et préférées de l'instrument selon l'invention sont définies dans les revendications dépendantes.

L'invention sera décrite plus en détail ci-après à l'aide de forme d'exécutions illustrées dans le dessin, dans lequel représente:
- Fig. 1: en vue générale d'un instrument porte-outil selon l'invention, avec les composantes d'entraînement et de verrouillage en position non bloquée;
- Fig. 2: une vue analogue à celle de la Fig. 1 avec les composantes en position verrouillée;
- Fig. 3: une vue en perspective d'un détail de l'instrument (tête porte- outil), partiellement en coupe;

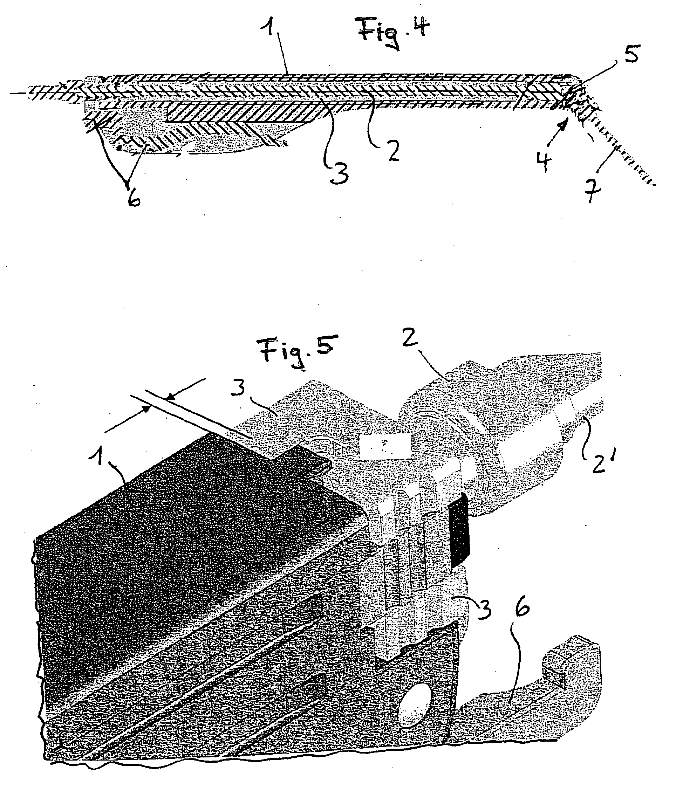

- Fig. 4: l'instrument porte-outil en coupe schématique;
- Fig. 5: une vue en perspective agrandie d'un autre détail (partie arrière de l'instrument avec mécanisme de blocage à genouillère en position ouverte, et
- Fig. 6a - 6d: le mécanisme de blocage à genouillère, en partie en coupe, en positon ouverte et fermée respectivement.

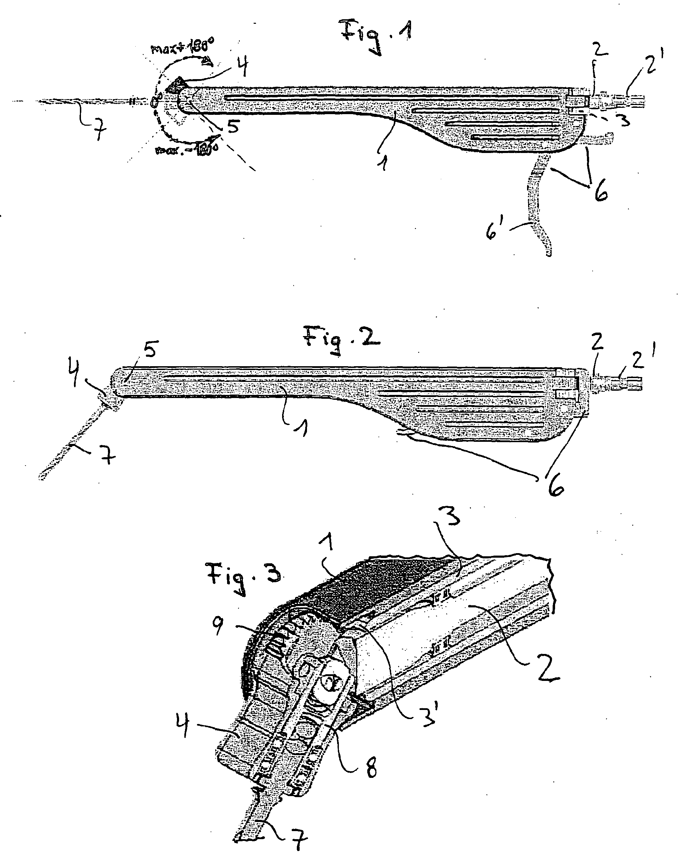

La Fig. 1 du dessin montre en vue latérale et très schématiquement un instrument porte-outil selon l'invention, comprenant un boitier 1 en forme de poignée longitudinale et logeant à l'intérieur un arbre d'entraînement et un tube de blocage 3 (Fig 3) qui mènent de l'extrémité arrière le long d'un axe longitudinal jusqu'a une tête porte-outil 4 à l'avant de l'instrument.

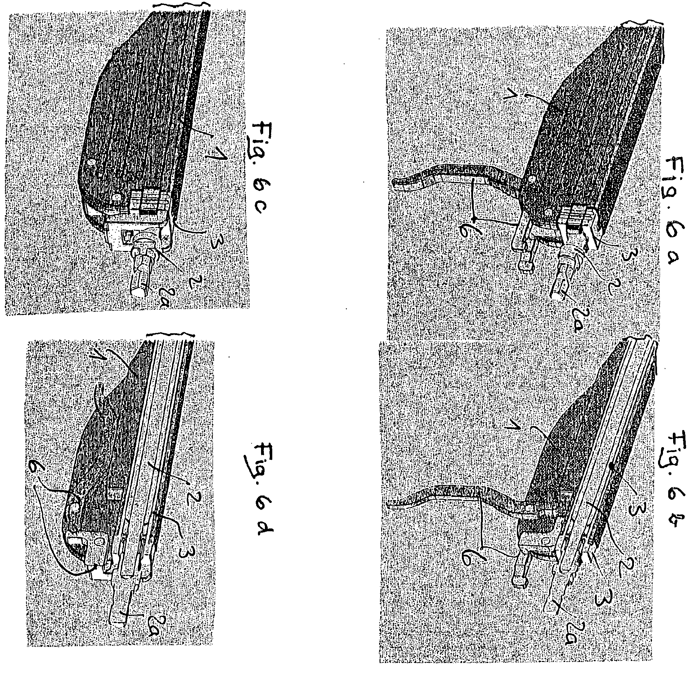

L'arbre d'entraînement 2 et le tube de blocage 3 peuvent être démontés de l'instrument en les tirant hors du boitier vers l'arrière ou être bloquées en position de travail au moyen d'un mécanisme à genouillère 6 (détail voir Fig. 6a - 6d).

La tête porte-outil 4 peut être orientée angulairement autour d'un axe fixe 5 de ± 180° au maximum à partir de la positon neutre 0° (dirigé selon l'axe longitudinal de l'instrument) et est bloquée dans toute positon choisie au moyen dudit tube de blocage 3 (voir détail Fig. 3).

La Fig. 2 montre l'instrument avec les composantes d'entraînement et de blocage 2 resp. 3 bloquées en direction longitudinale par le mécanisme à genouillère 6 avec la tête porte-outil équipée d'un outil (foret) 7.

L'arbre d'entraînement 2, qui à l'extrémité arrière 2' peut être connecté à un moteur (non représenté), est connecté en positon de travail à son extrémité avant à une partie porte-outil 8 pour un outil rotatif 7 logée dans la tête porte-outil 4.

L'accouplement entre l'arbre d'entraînement 2 et ladite partie porte-outil 8 peut être d'une construction quelconque, par exemple du type joint universel, joint de cardan, roue dentée ou autre. La construction du mécanisme d'accouplement permettant une orientation de ± 180° sera décrite dans un brevet séparé et ne forme pas partie de la présente invention.

Le tube de blocage 3 en position de travail (blocage de la tête porte-outil 4) s'engrène dans une partie dentée 9 sur
la tête porte-outil 4 et maintient cette tête dans la position choisie avant d'avoir été bloquée.

L'instrument perceur renvoi d'angle à tête orientable permet lors de sa connexion à un moteur (accouplement stryker, hudson, AO ou autres) la transmission d'un couple.

En connectant différents ancillaires 7 sur le devant de sa tête orientable 4, il autorise, le perçage, le fraisage, le taraudage, l'alésage des différentes parties osseuses lors d'une intervention chirurgicale de type orthopédique. Le système permet lors de la connexion de tournevis à l'instrument, le vissage de diverses vis implantables ou non implantables telles que des vis six pans, torx, cruciformes ou autres.

La principale innovation réside dans le fait que la tête porte-outil 4 est orientable et peut couvrir un rayon d'action allant théoriquement de + 180° à - 180° en passant par 0°.

Il permet de couvrir un rayon d'action que les perceurs à renvoi d'angles fixe (ex. 45°) ne peuvent garantir. De ce fait il peut être utilisé dans une multitude de techniques opératoires de type orthopédiques couvrant les chirurgies du genou, de la hanche, de la traumatologie, de la colonne vertébrale, de l'épaule, des extrémités ou de maxillo-facial, où les anatomies osseuses requièrent différentes approches ou accès.

De par sa conception, la partie proximale de l'instrument est de faible encombrement, et autorise les techniques mini invasives.

Le fait que la tête 4 de l'instrument soit orientable, permet ainsi d'atteindre certaines cavités osseuses ou accès difficiles qui ne pourraient être atteints que par plusieurs instruments classiques combinés. Pour exemple, nous pouvons citer dans la traumatologie, les réductions de fractures par pose de plaques qui traditionnellement peuvent combiner l'utilisation de divers guides et flexibles lors des perçages et taraudages des passages de vis. L'instrument pourra dans certains cas se substituer à cette combinaison de deux instruments et ainsi faciliter la technique.

L'indexage de la position de la tête se fait par un mouvement simple de l'abaissement du levier 6'. Celui-ci libère la genouillère et permet le recule du tube 3. Celui-ci libère la denture 9 de la tête 4 qui de ce fait, peut être orientée sous l'angle choisi par le praticien et, correspondant à l'application chirurgicale choisie. Une fois l'angle choisi, le tube 3 est poussé afin de bloquer la denture 9 de la tête 4 et sa position. Le levier 6' doit être mis en positon fermée afin qu'il transmette un pression constante sur la genouillère qui elle-même, en appuyant sur le tube 3 verrouille la positon de la tête 4.

Dans cette positon l'instrument peut-être utilisé en rotation sous l'angle choisi.

L'instrument peut également être complètement démonté afin d'effectuer un nettoyage approfondi avant la stérilisation.

Pour démonter 1 instrument, il s'agit du même mécanisme que celui qui permet d'orienter et de verrouiller la position de l'angle de la tête 4. Le mécanisme de démontage de l'instrument est très simple d'utilisation et un minimum de mouvements est nécessaire afin de réaliser l'opération de démontage. Il suffit d'abaisser le levier 6' qui libère la genouillère et de positionner la tête 4 à 0°. Puis retirer l'arbre de transmission 2 puis le tube 3. Une fois l'appareil démonté, l'utilisateur se retrouvera avec trois morceaux d'instrument (ancillaire exclus), soit: le corps principal 1, l'arbre de transmission 2 et le tube 3. Ces trois éléments séparés remplissent de manière efficace les exigences et les directives relatives à la décontamination et au nettoyage et à la stérilisation des instruments chirurgicaux. Le remontage de l'instrument s'effectue dans l'ordre inverse des opérations ci-dessus.

La forme et les rainures latérales sur le boitier 1 ont été étudiées pour permettre la fixation de guides afin d'adapter et d'utiliser le produit dans les techniques de chirurgies assistées par navigation. Ces rainures permettent également de fixer une poignée latérale coulissante afin de gagner en stabilité lors de l'utilisation de l'instrument.

## Revendications

1. Instrument porte-outil en forme de poignée longitudinale, notamment pour applications chirurgicales, avec un arbre d'entraînement (2) pour un outil (7), cet arbre (2) pouvant être glissé le long d'un axe longitudinal dans l'instrument à partir de l'extrémité arrière de l'instrument pour être connecté à la partie porte-outil (8) d'un outil rotatif logée dans une tête porte-outil (4) montée à l'extrémité avant de l'instrument, la tête porte-outil (4) logeant ladite partie porte-outil (8) est montée à l'extrémité avant de l'instrument de manière orientable angulairement par rapport à l'axe longitudinal de l'instrument autour d'un axe de pivotement fixe transversal à cet axe, des moyens (3', 9) étant prévus pour bloquer la tête orientable dans toute position angulaire choisie, lesdits moyens comprennent une partie (9) de ladite tête (4) **caractérise en ce que** lesdits moyens comprennent un tube (3) à introduire dans l'instrument à partir de l'extrémité arrière de l'instrument et entourant ledit arbre d'entraînement (2), le bord avant (3') dudit tube (3) étant destiné à entrer en contact avec ladite partie (9) de ladite tête (4) pour bloquer cette dernière dans une position présélectionnée.

2. Instrument selon la revendication 1, comprenant un jeu entre ledit tube (3) et ledit arbre d'entraînement (2) permettant le mouvement relatif libre entre ces deux éléments, dit tube (3) et dit arbre (2) pouvant être montés et démontés individuellement.

3. Instrument selon la revendication 2, **caractérise par** des moyens (6) pour bloquer ledit tube (3) et l'arbre d'entraînement (2) contre un déplacement axial dans leurs
positons de travail, c'est-a-dire en contact de blocage avec la tête (4) respectivement connectée avec ladite partie porte-outil (8) pour entraîner cette dernière.

4. Instrument selon la revendication 3, **caractérise en ce que** les moyens de blocage (6) comprennent un mécanisme à genouillère dont les éléments d'arrêt en positon de blocage s'appliquent contre l'arrière dudit tube (3) et le cas échéant contre une partie arrière de l'arbre d'entraînement.

## Claims

1. A tool-holder instrument in the form of a longitudinal handle, particularly for surgical applications, with a drive shaft (2) for a tool (7), which shaft (2) can be slid along a longitudinal axis in the instrument from the back of the instrument and be connected to the tool-holder part (8) of a rotary tool housed in a tool-holder head (4) mounted on the front of the instrument, the tool-holder head (4) housing said tool-holder part (8) is mounted on the front of the instrument in such a way that it can be moved at an angle in relation to the longitudinal axis of the instrument around a set swiveling axis transverse to that axis; means (3', 9) are provided to lock the moveable head into any angular position chosen, said means comprise a part (9) of the head (4) **characterized in that** said means comprise a tube (3) to be inserted into the instrument from the back of the instrument surrounding the drive shaft (2), the front edge (3') of said tube (3) having the destination to be in contact with said part (9) of the head (4) to lock the head in a pre-selected position.

2. The instrument in claim 1, comprising a play between said tube (3) and said drive shaft (2) allowing free relative movement between these two elements; said tube (3) and said shaft (2) can be assembled and disassembled individually.

3. The instrument in claim 2, **characterized by** means (6) of locking said tube (3) and the drive shaft (2) against axial displacement in their working positions, that is, in locking contact with the head (4), respectively connected to said tool-holder part (8) to drive the latter.

4. The instrument in claim 3, **characterized by** the fact that the locking means (6) include an articulated mechanism whose stopping elements in the locking position are applied against the back of said tube (3) and, if need be, against the back of the drive shaft.

## Patentansprüche

1. Ein Werkzeughalterinstrument in der Form eines längsförmigen Handgriffs, insbesondere für chirurgische Anwendungen, mit einer Antriebswelle (2) für ein Werkzeug (7), welche Welle (2) entlang einer länglichen Achse in dem Instrument von der Rückseite des Instruments entlanggleiten kann und welche zu dem Werkzeughalterteil (8) eines rotierenden Werkzeugs in einem Werkzeughalterkopf (4), der auf der Vorderseite des Instruments befestigt ist, verbunden werden kann, der Werkzeughalterkopf (4), der besagten Werkzeughalterteil (8) aufnimmt, ist auf der Vorderseite des Instruments in einer Weise befestigt, dass es in einem Winkel in Bezug auf die längliche Achse des Instruments um eine gegebene Schwenkachse quer zu dieser Achse bewegt werden kann; Mittel (3', 9) werden bereitgestellt, um die beweglichen Kopf in irgendeiner gewählten Winkelposition zu arretieren, besagte Mittel umfassen einen Teil (9) des Kopfes (4), **gekennzeichnet dadurch, dass** besagte Mittel ein Rohr umfassen, welches in das Instrument von der Rückseite des Instruments eingefügt wird, und die Antriebswelle (2) umgibt, die Vorderseite (3') des Rohrs (3) hat den Zweck in Kontakt mit besagtem Teil (9) des Kopfes zu sein, um den Kopf in einer vorgewählten Position zu arretieren.

2. Das Instrument gemäss Anspruch 1, umfassend ein Spiel zwischen besagtem Rohr (3) und besagter Antriebswelle (2), was eine freie, relative Bewegung zwischen diesen Elementen erlaubt; besagtes Rohr (3) und besagte Welle (2) können einzeln zusammen- und auseinandergebaut werden.

3. Das Instrument gemäss Anspruch 2, **gekennzeichnet durch** Mittel (6) zum Arretieren (6) des besagten Rohrs (3) und des Antriebsschafts (2) gegen axiale Verschiebung in ihren Arbeitspositionen, d.h. **durch** Arretieren des Kontakts mit dem Kopf (4) respektive verbunden mit besagten Werkzeughalterteil (8), um den letzteren anzutreiben.

4. Das Instrument gemäss Anspruch 3, **gekennzeichnet dadurch, dass** die Arretierung (6) einen Gelenkmechanismus enthalten, dessen Stoppelemente in der Verschlussposition gegen die Rückseite des Rohrs (3) wirken, und, wenn nötig, gegen die Rückseite der Antriebswelle.
